# EUROPEAN PATENT SPECIFICATION

(11) **EP 1 885 690 B1**
(45) Date of publication and mention of the grant of the patent: **06.10.2010**
(21) Application number: 06744875.3
(22) Date of filing: 05.05.2006
(51) Int. Cl.: C07C 253/32, C07C 255/23

(54) **PROCESS FOR PREPARATION OF ALKYL AND ALCOXYALKYL-ALPHA -CYANOACRYLATES BY DEPOLYMERISATION OF POLY(ALKYL-ALPHA CYANOACRYLATES) OR POLY(ALCOXYALKYL-ALPHA -CYANOACRYLATES) AND ITS USAGE AS TECHNICAL AND/OR MEDICAL ADHESIVE**
VERFAHREN ZUR HERSTELLUNG VON ALKYL- UND ALKOXYALKYL-ALPHA-CYANOACRYLATEN DURCH DEPOLYMERISIERUNG VON POLY(ALKYL- ALPHA-CYANOACRYLATEN) BZW. POLY(ALKOXYALKYL-ALPHA-CYANOACRYLATEN) UND DESSEN VERWENDUNG ALS INDUSTRIELLER UND/ODER MEDIZINISCHER KLEBSTOFF
PROCÉDÉ DE PREPARATION D'ALFA-CYANOACRYLATES D'ALKYLES ET D'ALCOXYALKYL PAR DEPOLYMERISATION DE POLY(ALFA-CYANOACRYLATED D'ALKYLES ET D'ALCOXYALKYL) OU DE POLY(ALPHA-CYANOACRYLATES) ET SA UTILISATION COMME ADHESIFS TECHNIQUES ET/OU MEDICAUX

(30) Priority: 06.05.2005 PT 10327205
(43) Date of publication of application: 13.02.2008
(73) Proprietor: Universidade do Minho, 4700-320 Braga (PT)
(72) Inventor: ZLATEV DÉNCHEV, Zlatan, Universidade do Minho, P-4800-058 Guimarães (PT); IVANOVA TÓMANOVA, Milena, Universidade do Minho, P-4800-058 Guimarães (PT); MAGALHÃES DA CUNHA, António, Universidade do Minho, P-4800-058 Guimarães (PT)
(74) Representative: Ferreira, Maria Silvina
(86) International application number: PCT/IB2006/051426
(87) International publication number: WO 2006/120628

(56) References cited:
- DE-A1- 19 921 484
- US-A- 3 254 111
- GUSEVA, SENCHENYA, MAGER, TSYRYAPKIN, GOLOLOBOW: "Synthesis of functionally substituted 2-cyanoacrylates" RUSSIAN CHEMICAL BULLETIN, vol. 43, no. 2, 1994, pages 595-598, XP009072190

## Description

### Field of the invention

This invention relates to the preparation of α-cyanoacrylates in monomeric form and is particularly concerned with the depolymerisation of poly(alkyl-α-cyanoacrylates) or poly(alcoxyalkyl-α-cyanoacrylates) (PCA) to produce monomers applicable in technical or medical adhesives. More specifically, the invention discloses an improved method for controlled depolymerisation of polymeric cyanoacrylates in the presence of a depolymerisation system containing specified amounts of phosphorous pentoxide (P₂O₅), hydroquinone, *ortho*-phosphoric acid and *para*-toluenesulfonic acid. The depolymerisation method disclosed allows the attainment of high-yield and high-purity alkyl or alcoxyalkyl-α-cyanoacrylates in reactors of simple design, with a minimum quantity of liquid waste and without the use of hazardous gases.

### Background of the invention

Cyanoacrylate monomers, when applied as a thin layer between two surfaces made out of similar or different materials, e.g. metals, polymers (with the exception of polyolefins), wood, stone, living tissue, etc. are able to bond to them rapidly and without the use of heat or catalysts. Conventionally, the esters of the α-cyanoacrylates are prepared using a two-stage reaction. In the first stage, the corresponding cyanoacetate is made to react with formaldehyde (as gas, aqueous solution or in polymeric form) in a basic environment to form poly(alkyl-α-cyanoacrylates). In the second stage, the polymer is cracked (i.e., depolymerised), purified and stabilized to obtain the cyanoacrylate adhesive.

The above reaction scheme was proposed for the first time by Ardis in U.S. Pat. No 2,467,927, which discloses the condensation of formalin (ca. 40 wt. % of formaldehyde in water) with alkyl cyanoacetate using basic catalysts. The polymer thus formed is depolymerised in the presence of catalytic amounts of P₂O₅, employing a gaseous inhibitor of the back polymerization - sulphur dioxide SO₂ or nitric oxide NO. Later US patent No 2,721,858 by Joyner and Hawkins discloses the substitution of formalin, in the stage of condensation, with a formaldehyde polymer and the combined use of phosphorus pentoxide and hydroquinone in the stage of depolymerisation. Stable monomers were only obtained when SO₂-gas was passed through the reactor during the depolymerisation stage.

US patent No 2,756,251 by Joyner and Shearer describes a similar depolymerisation system comprising phosphorous pentoxide and hydroquinone (polymerization inhibitors for the liquid cyanoacrylate). The SO₂-gas is equally used to inhibit the spontaneous polymerization of the cyanoacrylate vapours. The use of high-boiling tertiary phosphoric acid esters as reaction media for depolymerisation, e.g., tricresyl phosphate, was here proposed for the first time. These phosphate esters should have boiling temperatures about 60-80°C higher than the depolymerisation temperature. The amount of tricresyl phosphate required in the process disclosed in this patent is about 0.9 times that of the polymeric raw material, thus creating significant amounts of liquid waste. The reaction is conducted as a batch process.

An analogous method for making alkyl-α-cyanoacrylates was proposed by Robert Rabinowitz in US patent No 3,444,233 disclosing continuous depolymerisation of poly(alkyl-α-cyanoacrylate) esters. The polymer is first admixed with an inert liquid of high boiling point, such as tricresyl phosphate adding one or more polymerization inhibitors, such as phosphorus pentoxide and hydroquinone. This mixture is then depolymerised by heating a thin layer of this mixture in vacuum to produce high-purity monomeric α-cyanoacrylate vapours. The continuous process disclosed in this patent requires the use of large amounts of tricresyl phosphate (1.45 times the amount of the polycyanoacrylic acid esters), thus resulting in serious liquid pollution and disposal problems. Again, to get stable monomeric alkyl cyanoacrylates useful as instant adhesives, the depolymerisation has to be carried out in a stream of the hazardous and corrosive SO₂-gas.

US patent 5,436,363 by Wang et al. discloses a continuous process for making cyanoacrylates, which utilizes a thin-layer evaporator and a two-condenser heat transfer system. A poly(alkyl-α-cyanoacrylate) feed containing catalytic amounts of P₂ O₅ and hydroquinone without a high-boiling medium is introduced into a thin-film evaporator so as to carry out the depolymerisation reaction. The monomeric vapours produced by this process, where no high-boiling medium is used, do not require stabilization by SO₂-gas. They are then subjected to a two-stage heat transfer process for condensation and purification. The first stage involves a high temperature condenser operating at ca. 150°C that collects a fraction containing primarily 'dimers' of cyanoacrylate (i.e., dicyanoglytarates). Subsequently, the gaseous phase enters a low-temperature condenser, operating at -8°C, where the cyanoacrylate monomer is collected. The advantages of this scheme employing thin layer evaporator and an intermediate condenser are: (i) the elimination of the need of using a high-boiling heat transfer medium; (ii) the elimination of the use of hazardous gases (SO₂ or NO); (iii) the attainment of a high-yield and high-purity final product of cyanoacrylate mo nomers. The major disadvantages of the proposed process are related to: (i) a quite complex reactor design; (ii) the need for a continuous, large-scale production, which is not always appropriate when smaller volumes of monomeric cyanoacrylates are needed, as in the case of medical adhesives.

Other reaction schemes, not including condensation or depolymerisation processes, are also worth mentioning:
1. the protection of the cyanoacrylate monomer in the condensation stage, by formation of its anthracene Diels-Alder adduct, with subsequent cyanoacrylate liberation, involving the reaction of the adduct with maleic anhydride, avoiding the stage of depolymerisation and originating stable alkyl-cyanoacrylates, useful as industrial adhesives, as exemplified by U.S. Patents No. 3,463,804 by Ray and Doran and 4,012,402 (Buck);
2. the transesterification of the cyanoacrylate monomers with alcohols, as described in USSR authorship certification No 726086 by Y. B. Vojtekunas et al.;
3. the direct esterification of cyanoacrylic acid with alcohols is reported in German patent 3,415,181 by Schülter and Marten;
4. the thermal decomposition of alkyl 2-cyano-3-alkoxypropionates and the 3-acyloxy analogues are described in U.S. Patent No 2,467,926 by Ardis;
5. the pyrolysis of the cyanohydrin acetates of pyruvic acid esters is described in U.S. Pat. No 2,391,251 by Long.

The major disadvantages of all these processes are their high complexity, requiring the isolation and the purification of many intermediates and their very long duration (typically 10-16 h per batch), which renders them unsuitable for industrial applications. Especially inappropriate for the synthesis of medical adhesives is the use of Diels-Alder protection that requires the implementation of readily sublimating carcinogenic compounds with condensed benzene rings that can migrate into the final adhesive and enter in contact with living tissue.

### Summary of the invention

The present invention discloses a versatile process for the production of alkyl and alkoxyalkyl-α-cyanoacrylates, applicable on technical and medical adhesives, by introducing a new stabilization system at the stage of depolymerisation. The general formula of these compounds is the following: 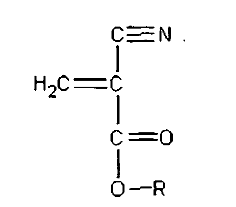 General formula of the alkyl or alkoxyalkyl-α-cyanoacrylates R = alkyl C₁-C₁₆ or alkoxyalkyl residue.

The new process proposed is simple in terms of reactor design, does not involve the generation of much liquid waste and excludes the use of hazardous inhibition gases.

### Detailed description of the invention

The new process disclosed in the present patent makes use of the aforementioned condensation-depolymerisation scheme, employing a new stabilization system in the stage of depolymerisation, which comprises phosphorus pentoxide, hydroquinone, *ortho*-phosphoric acid and *para*-toluenesulfonic acid, in specific concentrations, as indicated hereafter. The co-inventors have identified the strong stabilization effect of the said four-component system, eliminating significantly the undesired back polymerization of the monomeric cyanoacrylates (in both liquid and gaseous state) in the depolymerisation stage. Thus, the polymer phase remains less viscous and better depolymerisable during the entire depolymerisation, without the necessity of use of high-boiling solvents. Furthermore, the cyanoacrylate vapours do not polymerize spontaneously, avoiding the use of gaseous polymerization inhibitors, such as SO₂. It is an advantage of the process proposed that it allows the use of a one-condenser batch reactor of a versatile and simple design, useful for the small- and medium-scale production of more sophisticated cyanoacrylate monomers applied as medical adhesives. Another advantage of the process proposed is the fact that it does not require the use of non-recyclable, high-boiling point solvents (such as tricresyl phosphate) and/or dangerous, corrosive gases (SO₂) which required special safety and purification procedures.

According to the process of the present invention, the poly(alkyl- a -cyanoacrylate) or poly(alkoxyalkyl- a -cyanoacrylate) condensation product is mixed with a depolymerisation system comprising phosphorous pentoxide (P₂O₅), hydroquinone, *ortho -* phosphoric acid (crystalline or liquid) and *para*-toluenesulfonic acid in a one condenser batch reactor, heated within the range of 100-300°C, preferably 150-250°C and more preferably 180-220°C, and operating under a vacuum within the limits of 50-0.5 Torr (7x10⁻³ - 7x10⁻⁵ MPa), preferably 20-1 Torr (3x10⁻³ - 1.3x10⁻⁴ MPa) and more preferably 10-5 Torr (1.3x10⁻³ - 7x10⁻⁴ MPa), both depending on the structure of the monomer to be prepared.

Also depending on the structure of the monomer to be prepared, the relation between the phosphorous pentoxide and the hydroquinone is in the range, wt. parts, from 1:10 to 10:1, preferably between 1:1 and 1:5, more preferably between 1:1 and 1:3, and the relation between the *ortho*-phosphoric acid and *para*-toluenesulfonic acid is in the range from, wt. parts, 20:1 and 1:20, preferably between 15:1 and 10:1.

The present invention will now be described more specifically with reference to some typical examples. It is to be noted that the following examples are presented herein for the purpose of illustration and description and are not intended to be exhaustive or to limit the invention to the precise form of the present description.

### Example 1

160 parts (1.4 mol) of ethyl cyanoacetate, 170 parts of toluene, 20 parts of paraformaldehyde and 0.5 parts of piperidine were mixed and heated in a 0.5 litre , stirred reaction flask, fitted with a thermometer, condenser and a Dean-Stark trap. The mixture was refluxed while stirring until removing the theoretical amount of condensation water. Thereafter, the reaction vessel containing toluene solution of poly(ethyl-α-cyanoacrylate) was cooled down to room temperature. 4 parts of fresh phosphorus pentoxide and 1.5 parts of hydroquinone (weight ratio of 2.67:1.00), as well as 1 part of *para*-toluenesulfonic acid and 10 parts of *ortho*-phosphoric acid (weight ratio of 1:10) were then added with good stirring. The toluene solvent was removed under reduced pressure and the residue depolymerised by heating up to 180°C under a vacuum of 2 MPa (15 Torr) The distillate (102 parts) was collected in a cold flask containing 0.1 part sulphuric acid and 0.05 part of hydroquinone and subjected to additional vacuum distillation to get 94 parts of high-purity (+99%, GC) ethyl-α-cyanoacrylate. ***Example 2***

Example 1 is repeated except that 120 parts of butyl cyanoacetate were mixed with 23 parts of paraformaldehyde, 0.5 parts of piperidine in 150 parts of toluene using the same reaction vessel and conditions. Depolymerisation at 190°C under a vacuum of 1.33 MPa (10 Torr) was performed in the presence of 3 parts of P₂O₅, 2 parts of hydroquinone (weight ratio 1.5:1.0), 1 part of *para*-toluenesulfonic acid and 10 parts of *ortho*-phosphoric acid (weight ratio 1:10). After purification by additional vacuum distillation, 65 parts of high-purity (+99%, GC) and stabilized butyl-α-cyanoacrylate were obtained.

### Example 3

Example 1 is repeated except that 180 parts of 2-ethoxyethyl cyanoacetate were mixed with 32 parts of paraformaldehyde, 0.8 parts of piperidine in 200 parts of toluene using the same reaction vessel and conditions. The resulting poly(ethoxyethyl-α-cyanoacrylate) was depolymerised at 210°C under a vacuum of 0.67 MPa (5.0 Torr) in the presence of 6 parts of P₂O₅, 3 parts of hydroquinone (weight ratio of 2:1), 0.5 parts of *para*-toluenesulfonic acid and 5 parts of *ortho*-phosphoric acid (weight ratio of 1:10). After purification by additional vacuum distillation, 62 parts of high-purity (+98%, GC) and stabilized 2-ethoxyethyl-α-cyanoacrylate were obtained.

### References Cited

- Alan E. Ardis, to Goodrich B. F. Co., NY: "Preparation of monomeric alkyl-α-cyanoacrylates", US patent 2,467,927 (April 19, 1949).
- Frederick B. Joyner and Gary F. Hawkins, to Eastman Kodak Co, NY: "Method of making of α-cyanoacrylates", US patent 2,721,858 (October 25, 1955).
- Frederick B. Joyner and Newton H. Shearer Jun., to Eastman Kodak Co., NY: "Preparation of monomeric α-cyanoacrylates", US patent 2,756,251 (July 24, 1956).
- Robert Rabinowitz, to American Cyanamid Co, Maine: "Preparation of alkyl- and aryl-α-cyanoacrylates", US patent 3,444,233 (May 13, 1969).
- Tien-Lu Wang, Tso-Chi Chiu, Kun-Chuo Chen, to Industrial Technology Research Institute, Chutung, Taiwan: "Method for making of alkyl-α-cyanoacrylates from polyalkyl-α-cyanoacrylates", US patent No 5,436,363, (July 25, 1995).
- Neil Hunter Ray and Peter Doran, to ICI Ltd., UK : 'Preparation of α-cyanoacrylic esters', US patent 3,463,804 ( August 26, 1969).
- Carl J. Buck, to Johnson & Johnson , NY , 'Modified cyanoacrylate monomers and methods for preparation', US patent 4,012,402 (March 15, 1977).
- Y. B. Vojtekunas, A. M. Polyakova, K. A. Mager, Y. B. Kohanov, A. I. Vojtkov, to Institute of Organometallic compounds, USSR Academy of Sciences, USSR, 'Method of making esters of the α-cyanoacrylic acid', USSR authorship certification 726,086 (March 1980) (In Russian).
- Kaspar Schülter and Klaus Marten, to Henkel KGaA, BRD, 'α-Cyanacrylsäure', Offenlegungsschrift DE 3415181 A1 (October 31, 1985 ) (in German).
- Alan E. Ardis, to Goodrich B. F. Co., NY: 'Preparation of monomeric alkyl-α-cyanoacrylates', US patent 2,467,926 ( April 19, 1949 ).
- John B. Long, to Wingfoot Co., Delaware , 'Derivatives of fatty acids and method of preparing same', US patent 2,391,251 ( December 18, 1945 ).

## Claims

1. The process for the preparation of alkyl or alcoxyalkyl-α-cyanoacrylates of general formula (I) 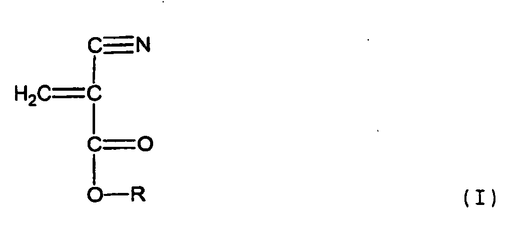 wherein R is a C₁-C₁₆-alkyl or alkoxyalkyl residue **characterized by** comprising the depolymerisation of the corresponding poly(alkyl-α-cyanoacrylate) or poly(alkoxyalkyl-α-cyanoacrylate) in the presence of a multi-component stabilizing system, which comprises
(a) phosphorus pentoxide;
(b) hydroquinone;
(c) *ortho*-phosphoric acid; and
(d) *para*-toluenesulfonic acid.
wherein the relation between the phosphorous pentoxide and the hydroquinone is in the range, wt. parts, from 1:10 to 10:1 and the relation between the *ortho*-phosphoric acid and the *para-*toluenesulfonic acid being in the range from, wt. parts, 20:1 and 1:20, the depolymerisation step being performed in the range of temperatures between 100 and 300°C and being carried out in vacuum in the range from 6.67.10³ to 66.7 Pa (50 to 0.5 Torr).

2. A process according to claim 1, **characterized by** the relation between the phosphorous pentoxide and the hydroquinone being in the range, wt. parts, from 1:1 and 1:5.

3. A process according to claims 1 and 2, **characterized by** the relation between the phosphorous pentoxide and the hydroquinone being in the range, wt. parts, from between 1:1 and 1:3.

4. A process according to claim 1, **characterized by** the relation between the *ortho*-phosphoric acid and the *para-*toluenesulfonic acid being in the range from, wt. parts, between 15:1 and 10:1.

5. A process according to claim 1, **characterized by** said alkyl-α-cyanoacrylate being ethyl-α-cyanoacrylate and said poly(alkyl-α-cyanoacrylate) being poly(ethyl-α-cyanoacrylate).

6. A process according to claim 1, **characterized by** said alkyl-α-cyanoacrylate being butyl-α-cyanoacrylate, and said poly(butyl-α-cyanoacrylate) being poly(ethyl-α-cyanoacrylate).

7. A process according to claims 1, **characterized by** said alkoxyalkyl-α-cyanoacrylate being 2-ethoxyethyl-α-cyanoacrylate, and said poly(alkoxyalkyl-α-cyanoacrylate) being poly(2-ethoxyethyl-α-cyanoacrylate).

8. A process according to claim 1, **characterized by** the depolymerisation step being performed in the range of temperatures between 150 and 250°C.

9. A process according to claim 8, **characterized by** the depolymerisation step being performed in the range of temperatures between 180 and 220°C.

10. A process according to claim 9, **characterized by** the depolymerisation step being carried out in vacuum in the range from between 2.67 MPa and 133 Pa (20 and 1 Torr).

11. A process according to claim 1, **characterized by** the depolymerisation step being carried out in vacuum in the range from between 1.33 to 0.67 MPa (10 and 5 Torr).

12. The process according to claims 1 to 11 **characterized by** further comprising an additional step of vacuum distillation.

13. Use of the process, according to claims 1 to 12, **characterized** for being applied to the preparation of alkyl or alcoxyalkyl-α-cyanoacrylates of general formula (I).

14. Use of the process, according the claim 10, **characterized** for being applied to the preparation of technical or medical adhesives based on the alkyl or alcoxyalkyl-α-cyanoacrylates of general formula (I).

## Patentansprüche

1. Verfahren zur Herstellung von Alkyl- bzw. Alkoxy-Alky-α-Cyanakrylaten mit der allgemeinen Formel (I) 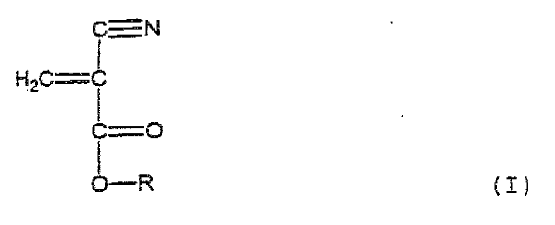 wobei R einen C₁-C₁₆-Alkyl- oder -Alkoxy-Alkyl-Rest ist, **dadurch gekennzeichnet, dass** es die Depolymerisation von den entsprechenden poly(Alkyl-α-Cyanoakrylaten) oder poly(Alkoxy-Alkyl-α-Cyanoakrylaten) in Gegenwart von einem mehrkomponentigen aus unten angegebenen Komponenten bestehenden Stabilisierungssystem beinhaltet:
(a) Phosphorpentoxid;
(b) Hydrochinon;
(c) *ortho*-Phosphorsäure; und
(d) *para*-Toluolsulfonsäure.
wobei das Verhältnis in Gewichtsteilen zwischen Phosphorpentoxid und Hydrochinon sich in dem Bereich von 1:10 bis zu 10:1 befindet und das Verhältnis in Gewichtsteilen zwischen *ortho*-Phosphorsäure und *para-*Toluolsulfonsäure sich in dem Bereich von 20:1 bis zu 1:20 befindet, und wobei die Depolymerisationsphase in dem Temperaturbereich von 100 bis zu 300 °C und in dem Vakuumbereich von 6,67.10³ bis zu 66,7 Pa (5C bis 0.5 Torr) stattfindet.

2. Verfahren nach Anspruch 1, **dadurch gekennzeichnet, dass** das Verhältnis in Gewichtsteilen zwischen Phosphorpentoxid und Hydrochinon sich in dem Bereich von 1:1 bis zu 1:5 befindet.

3. Verfahren nach Anspruch 1 oder 2, **dadurch gekennzeichnet, dass** das Verhältnis in Gewichtsteilen zwischen Phosphorpentoxid und Hydrochinon sich in dem Bereich von 1:1 bis zu 1:3 befindet.

4. Verfahren nach Anspruch 1, **dadurch gekennzeichnet, dass** das Verhältnis in Gewichtsteilen zwischen *ortho-*Phosphorsäure und *para*-Toluolsulfonsäure sich in dem. Bereich von 15:1 bis zu 10:1 befindet.

5. Verfahren nach Anspruch 1, **dadurch gekennzeichnet, dass** das oben erwähnte Alkyl-α-Cyanoakrylat ein Ethyl-α-Cyanoakrylat ist, und dass das oben erwähnte Poly(Alkyl-α-2yanoakrylat) ein Poly(Ethyl-α-Cyanoakrylat) ist.

6. Verfahren nach Anspruch 1, **dadurch gekennzeichnet, dass** das oben erwähnte Alkyl-α-Cyanoakrylat ein Butyl-α-Cyanoakrylat ist, und dass das oben erwähnte Poly(Buzyl-α-Cyanoakrylat) ein Poly(Ethyl-α-Cyanoakrylat) ist.

7. Verfahren nach Anspruch 1, **dadurch gekennzeichnet, dass** das oben erwähnte Alkyl-α-Cyanoakrylat ein 2-Ethoxyethyl-α-Cyanoakrylat ist, und dass das oben erwähnte Poly(Alkoxy-Alkyl-α-Cyanoakrylat) ein Poly(Ethyl-α-Cyanoakrylat) ist.

8. Verfahren nach Anspruch 1, **dadurch gekennzeichnet, dass** die Depolymerisationsphase in dem Temperaturbereich von 150 bis zu 250 °C stattfindet.

9. Verfahren nach Anspruch 8, **dadurch gekennzeichnet, dass** die Depolymerisationsphase in dem Temperaturbereich von 180 bis zu 200 °C stattfindet.

10. Verfahren nach Anspruch 9, **dadurch gekennzeichnet, dass** die Depolymerisationsphase in dem Vakuumbereich von 2,67 MPa bis zu 133 Pa (20 und 1 Torr) stattfindet.

11. Verfahren nach Anspruch 1, **dadurch gekennzeichnet, dass** die Depolymerisationsphase in dem Vakuumbereich von 1,33 MPa bis zu 0,67 MPa (10 und 5 Torr) stattfindet.

12. Das Verfahren nach Ansprüche 1 bis 11, **dadurch gekennzeichnet, dass** es außerdem eine weitere Phase der Vakuumdestillation beinhaltet.

13. Anwendung des Verfahrens nach Ansprüche 1 bis 12, **dadurch gekennzeichnet, dass** es für die Herstellung von Alkyl- oder Alkoxy-Alkyl-α-Cyanoakrylaten mit der allgemeinen Formel (I) benutzt wird.

14. Anwendung des Verfahrens nach Anspruch 10, **dadurch gekennzeichnet, dass** es für die Herstellung von technischen oder medizinischen Klebstoffen anhand von Alkyl- oder Alkoxy-Alkyl-α-Cyanoakrylaten mit der allgemeinen Formal (I) benutzt wird.

## Revendications

1. Procédé de préparation de cyanoacrylates d'alkyle ou de α-cyanoacrylates d'alkohyalkyle avec la formule générale (I) 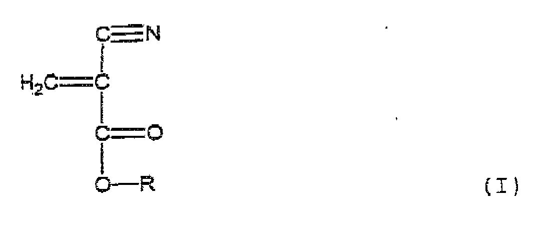 dont R est un résidu de C₁-C₁₆-alkyle ou d'alkoxyalkyle, **caractérisé en ce que** ce procédé comporte la dépolymérisation du poly(α-cyanoacrylate d'alkyle) ou du poly(α-cyanoacrylate d'alkoxyalkyle) correspondant en présence d'un système de stabilisation avec plusieurs composants, comprenant
(a) pentoxyde de phosphore;
(b) hydroquinone;
(c) acide *ortho*phosphorique ; et
(d) acide *para*toluènesulfonique.
étant donné que le rapport, en partie de poids, entre le pentoxyde de phosphore et l'hydroquinone est de 1:10 à 10:1 et le rapport, en partie de poids, entre l'acide *ortho*phosphorique et l'acide *para*toluènesulfonique est de 20:1 à 1:20, et **en ce que** l'étape de la dépolymérisation est effectuée dans la gamme de températures de 100 à 300 °C et dans la gamme de vide de 6,67.10³ à 66,7 Pa (50 à 0,5 Torr).

2. Procédé selon la revendication 1, **caractérisé en ce que** le rapport, en partie de poids, entre le pentoxyde de phosphore et l'hydroquinone est de 1:1 à 1:5.

3. Procédé selon les revendications 1 et 2, **caractérisé en ce que** le rapport, en partie de poids, entre le pentoxyde de phosphore et l'hydroquinone est de 1:1 and 1:3.

4. Procédé selon la revendication 1, **caractérisé en ce que** le rapport, en partie de poids, entre l'acide *ortho*phosphorique et l'acide *para*toluènesulfonique est de 15:1 à 10:1.

5. Procédé selon la revendication 1, **caractérisé en ce que** l'avant mentionné α-cyanoacrylate d'alkyle est un α-cyanoacrylate d'éthyle et **en ce que** l'avant mentionné poly(α-cyanoacrylate d'alkyle) est un poly(α-cyanoacrylate d'éthyle).

6. Procédé selon la revendication 1, **caractérisé en ce que** l'avant-mentionné α-cyanoacrylate d'alkyle est un α-cyanoacrylate de butyle et **en ce que** l'avant-mentionné poly(α-cyanoacrylate de butyle) est un poly(α-cyanoacrylate d'éthyle).

7. Procédé selon la revendication 1, **caractérisé en ce que** l'avant-mentionné α-cyanoacrylate d'alkoxyalkyle est un α-cyanoacrylate de 2-éthoxyéthyle et **en ce que** l'avant-mentionné poly(α-cyanoacrylate d'alkoxyalkyle) est un poly(α-cyanoacrylate de 2-éthoxyéthyle).

8. Procédé selon la revendication 1, **caractérisé en ce que** l'étape de la dépolymérisation est effectuée dans la gamme de températures de 150 à 250 °C.

9. Procédé selon la revendication 8, **caractérisé en ce que** l'étape de la dépolymérisation est effectuée dans la gamme de températures de 180 à 200 °C.

10. Procédé selon la revendication 9, **caractérisé en ce que** l'étape de la dépolymérisation est effectuée dans la gamme de vide de 2,67 MPa à 133 Pa (20 à 1 Torr).

11. Procédé selon la revendication 1, **caractérisé en ce que** l'étape de la dépolymérisation est effectuée dans la gamme de vide de 1,33 MPa à 0,67 Pa (10 à 5 Torr).

12. Procédé selon les revendications 1 à 11, **caractérisé en ce qu'**il comprenne aussi une étape supplémentaire de distillation à vide.

13. Utilisation du procédé selon les revendications 1 à 12, **caractérisé en ce qu'**il doit être appliqué à la préparation de α-ayanoaarylates d'alkyle ou de α-cyanoacrylates d'alkoxyalkyle avec la formule générale (I) .

14. Utilisation du procédé selon les revendications 10, **caractérisé en ce qu'**il doit être appliqué à la préparation de colles techniques ou médicales basées sur des α-cyanoacrylates d'alkyle ou des α-cyanoacrylates d'alkoxyalkyle avec la formule générale (I).
